# EUROPEAN PATENT APPLICATION

(11) **EP 1 022 030 A1**
(43) Date of publication of application: **26.07.2000**
(21) Application number: 99937016.6
(22) Date of filing: 10.08.1999
(51) Int. Cl.: A61K 45/00, A61K 31/557

(54) **PLASMINOGEN ACTIVATOR INHIBITOR 1 PRODUCTION REGULATORY AGENTS**

(30) Priority: 12.08.1998 JP 24102798
(71) Applicant: TORAY INDUSTRIES, INC., Tokyo 103-8666 (JP)
(72) Inventor: YAMAMOTO, Hiroshi, Kanazawa-shi, Ishikawa 921-8105 (JP); YAMAGISHI, Shoichi, Bronx, NY 10461 (US); KURUMATANI, Hajimu, Kamakura-shi, Kanagawa 248-0034 (JP); HISANO, Koichi, Tokyo 157-0063 (JP); HIRANO, Kazuo, Iruma-shi, Saitama 358-0012 (JP)
(74) Representative: Coleiro, Raymond
(86) International application number: JP9904331
(87) International publication number: WO0009161

(57) **Abstract**

Agents regulating the production of plasminogen activator inhibitor 1 which contain as the active ingredient intracellular cyclic AMP-augmenting substances.

## Description

### Technical Field

The present invention relates to a plasminogen activator inhibitor-1 production inhibitor which inhibits plasminogen activator inhibitor-1 production.

### Technical Background

Plasminogen activator inhibitor-1 (hereinafter sometimes referred to as PAI-1) is an inhibitor which inhibits tissue plasminogen activator (hereinafter sometimes referred to as t-PA). PAI-1 and t-PA are produced mainly by vascular endothelial cells and, by acting in a mutually competitive manner, regulate fibrinolysis. Specifically, t-PA has the action of converting plasminogen, which is the precursor of plasmin, to plasmin, and this plasmin converts fibrin, by degradation, to fibrin degradation products. On the other hand, because PAI-1 inhibits t-PA, it thus inhibits fibrin degradation. Hence, fibrinolysis regulation is carried out by t-PA and PAI-1 which are produced and secreted by vascular endothelial cells. Moreover, it has also been shown that PAI-1 is a urokinase-type plasminogen activator inhibitor produced by various cells in addition to endothelial cells.

In diabetes, the aggravation of arteriosclerosis and microvascular complications are regarded as causes of ischaemic heart disease, diabetic retinopathy and nephropathy, which are important complications of diabetes. PAI-1 is an important serine-protease inhibitor which weakens fibrin degradation. It has been shown as a result of many studies that a decrease in fibrinolytic activity due to increased plasma concentrations of PAI-1 is related to deep vein thrombosis, ischaemic heart disease and diabetic vascular disorders. In addition to a decrease in fibrinolytic activity, several other thrombotic abnormalities including hypercoagulability and platelet hyperaggregability are also exhibited by diabetic patients, and these contribute towards microthrombus formation and play an important role in the progress of diabetic microvascular disorders and diabetic macrovascular disorders. However, the molecular mechanisms of these abnormalities of thrombus formation are entirely unknown.

By reacting glucose non-enzymically with protein amino groups, reversible Schiff bases and then Amadori products can be formed. Via further complex reactions and rearrangements, these initial glycation products form irreversibly crosslinked fluorescent protein derivatives known as advanced glycation endproducts (AGE). In diabetes, it is known that AGE production and accumulation are greatly promoted in various tissues and in the plasma, and that this plays a role in the progress of diabetic vascular disorders.

Thus, since it has been shown that a reduction in fibrinolytic activity due to an increase in the plasma PAI-1 concentration is related to deep vein thrombosis, ischaemic heart disease and diabetic vascular disorders, it is thought that if compounds existed which could inhibit PAI-1 production, then they would be effective in the treatment and prevention of these diseases.

Consequently, the object of the present invention is to offer a plasminogen activator inhibitor-1 production inhibitor which can inhibit PAI-1 production.

### Disclosure of the Invention

As a result of painstaking research, the present inventors have discovered that intracellular cyclic AMP increasing substances inhibit PAI-1 production, and the present invention has been perfected based on this discovery.

Specifically, the present invention offers a plasminogen activator inhibitor-1 production inhibitor which contains, as the effective component, an intracellular cyclic AMP increasing substance.

### Brief Explanation of the Drawings

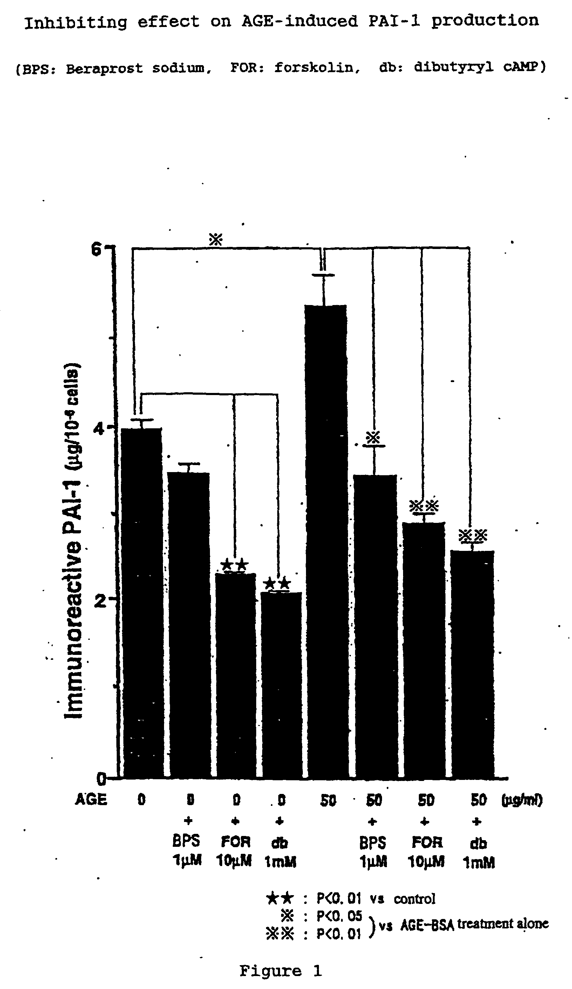

Figure 1 shows inhibition effectiveness in respect of AGE-induced PAI-1 production, that is to say, the amount of PAI-1 released into the medium per 10⁶ cells when vascular endothelial cells were treated with-various compounds at the illustrated concentrations.

### Optimum Form for Practising the Invention

As stated above, the PAI-1 production inhibitor of the present invention contains, as the effective component, a substance which brings about an increase in the amount of intracellular cyclic AMP (hereinafter referred to as cAMP).

The intracellular cAMP increasing substance employed in the present invention may be any substance provided that it can bring about an increase in the amount of cAMP within cells. As examples of preferred intracellular cAMP increasing substances, there are adenylate cyclase activators such as prostaglandin compounds and forskolin, cAMP derivatives such as dibutyryl cAMP which form cAMP within cells, and inhibitors of phosphodiesterases which degrade cAMP within cells, but there is no restriction thereto.

The aforesaid prostaglandin compounds may be any type of prostaglandin or derivative thereof having the prostanoic acid skeletal structure. Among these, the preferred prostaglandin compounds are PGI₂ derivatives, in particular, the 4,8-inter-m-phenylene prostaglandin I₂ derivatives which can be represented by the following general formula (I) 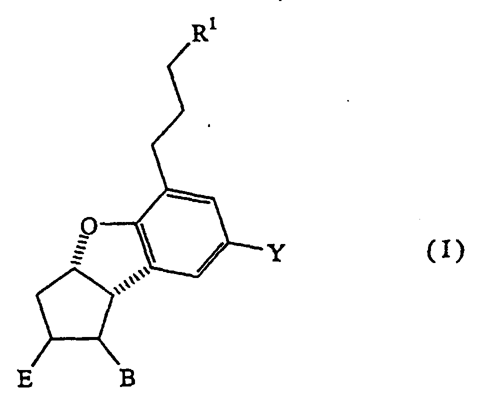 [where R¹ is
(A) COOR²
   (where R² is
   1) hydrogen or a pharmacologically acceptable cation,
   2) a C₁₋₁₂ straight chain alkyl or C₃₋₁₄ branched alkyl,
   3) -Z-R³
      (where Z is a valence bond or a straight chain or branched alkylene represented by CₜH₂ₜ, where t denotes an integer in the range 1-6, and R³ is a C₃₋₁₂ cycloalkyl or C₃₋₁₂ substituted cycloalkyl which is substituted with from one to three R⁴ groups, and R⁴ denotes hydrogen or a C₁₋₅ alkyl),
   4) -(CH₂CH₂O)ₙCH₃
      (where n denotes an integer in the range 1 to 5),
   5) -Z-Ar¹
      (where Z is defined as above and Ar¹ denotes phenyl, α-naphthyl, β-naphthyl, 2-pyridyl, 3-pyridyl, 4-pyridyl, α-furyl, β-furyl, α-thienyl, β-thienyl or substituted phenyl (where the substituent groups are at least one of chlorine, bromine, fluorine, iodine, trifluoromethyl, C₁₋₄ alkyl, nitro, cyano, methoxy, phenyl, phenoxy, p-acetamido-benzamido, -CH=N-NH-C(=O)-NH₂, -NH-C(=O)-Ph, - NH-C(=O)-CH₃ and -NH-C(=O)-NH₂)),
   6) -CₜH₂ₜCOOR⁴
      (where CₜH₂ₜ and R⁴ are as defined above),
   7) -CₜH₂ₜN(R⁴)₂
      (where CₜH₂ₜ and R⁴ are as defined above),
   8) -CH(R⁵)-C(=O)-R⁶
      (where R⁵ denotes hydrogen or benzoyl and R⁶ denotes phenyl, p-bromophenyl, p-chlorophenyl, p-biphenyl, p-nitrophenyl, p-benzamidophenyl or 2-naphthyl),
   9) -CₚH₂ₚ-W-R⁷
      (where W is -CH=CH-, -CH=CR⁷- or -C≡C-, and R⁷ is hydrogen or a C₁₋₃₀ straight chain or branched alkyl or aralkyl, and p denotes an integer in the range 1-5), or
   10) -CH(CH₂OR⁸)₂
      (where R⁸ denotes a C₁₋₃₀ alkyl or acyl),
(B) -CH₂OH
(C) -C(=O)N(R⁹)₂
   (where R⁹ represents hydrogen, a C₁₋₁₂ straight chain alkyl, C₃₋₁₂ branched alkyl, C₃₋₁₂ cycloalkyl, C₄₋₁₃ cycloalkylalkylene, phenyl, substituted phenyl (where the substituent groups are as defined in (A) 5) above), C₇₋₁₂ aralkyl or SO₂R¹⁰, and R¹⁰ represents a C₁₋₁₀ alkyl, C₃₋₁₂ cycloalkyl, phenyl, substituted phenyl (where the substituent groups are as defined in (A) 5) above), or a C₇₋₁₂ aralkyl, and the two R⁹ groups may be the same or different, but where one represents -SO₂R¹⁰ then the other R⁹ is not -SO₂R¹⁰), or
(D) -CH₂OTHP (where THP is a tetrahydropyranyl group),
   Y is hydrogen, a C₁₋₄ alkyl, chlorine, bromine, fluorine, formyl, methoxy or nitro,
   B is -X-C(R¹¹)(R¹²)OR¹³
      (where R¹¹ is hydrogen or a C₁₋₄ alkyl, R¹³ is hydrogen, C₁₋₁₄ acyl, C₆₋₁₅ aroyl, tetrahydropyranyl, tetrahydrofuranyl, 1-ethoxyethyl or t-butyl,
   X is
      1) -CH₂-CH₂-
      2) -CH=CH- or
      3) -C≡C- and
   R¹² represents
      1) a C₁₋₁₂ straight chain alkyl or C₃₋₁₄ branched alkyl,
      2) -Z-Ar²
         (where Z is as defined above and Ar² represents phenyl, α-naphthyl, β-napbthyl or phenyl substituted with at least one of chlorine, bromine, fluorine, iodine, trifluoromethyl, C₁₋₄ alkyl, nitro, cyano, methoxy, phenyl or phenoxy),
      3) -CₜH₂ₜOR¹⁴
         (where CₜH₂ₜ is as defined above and R¹⁴ represents a C₁₋₆ straight chain alkyl, C₃₋₆ branched alkyl, phenyl, phenyl substituted with at least one of chlorine, bromine, fluorine, iodine, trifluoromethyl, C₁₋₄ alkyl, nitro, cyano, methoxy, phenyl or phenoxy, cyclopentyl, cyclohexyl, or cyclopentyl or cyclohexyl substituted with from one to four C₁₋₄ straight chain alkyls),
      4) -Z-R³
         (where Z and R³ are as defined above),
      5) -CₜH₂ₜ-CH=C(R¹⁵)R¹⁶
         (where CₜH₂ₜ is as defined above, and R¹⁵ and R¹⁶ each independently represent hydrogen, methyl, ethyl, propyl or butyl), or
      6) -CᵤH₂ᵤ-C≡C-R¹⁷
         (where u is an integer in the range 1-7, CᵤH₂ᵤ represents a straight chain or branched alkylene, and R¹⁷ represents a C₁₋₆ straight chain alkyl),
   E represents hydrogen or -OR¹⁸ (where R¹⁸ represents a C₁₋₁₂ acyl, C₇₋₁₅ aroyl or R²(where R² is as defined above),
   and where the general formula represents the d-form, l-form or dl-form],
   or pharmacologically permissible salts thereof. Among these, Beraprost (the general name of (±)-(1R*,2R,3aS*,8bS*)-2,3,3a,8b-tetrahydro-2-hydroxy-1-[(E)-(3S*)-3-hydroxy-4-methyl-1-octen-6-inyl]-1H-cyclopenta[b]benzofuran-5-butyric acid) and its salts can be cited as particularly preferred.

The prostaglandin compounds which are used in the present invention can be produced by known methods, for example, the compounds represented by general formula (I) above can be produced by the method described in JP-B-1-53672.

Because the PAI-1 production inhibitor of the present invention can inhibit cellular PAI-1 production, it can increase the fibrin degrading action and fibrinogen degrading action due to plasmin. Thus, the PAI-1 production inhibitor of the present invention is effective in the prevention and treatment of ischaemic heart disease such as angina pectoris, myocardial infarction and cardiac insufficiency, ischaemic cerebrovascular disorders such as cerebral embolism, cerebral infarction and transient cerebral ischaemic attack, and various thromboses such as venous thrombosis of the vena cava and vena hepatica, pulmonary embolism and postoperative thrombosis, where thrombus formation is closely related to the onset and deterioration of the disease state. Moreover, it is particularly effective in the prevention and treatment of complications of diabetes comprising vascular disorders, microvascular disorders, diabetic neuropathy, retinopathy, nephropathy and ischaemic heart disease, where an increase in blood and accumulated AGE is closely related to an aggravation of the disease state.

The plasminogen activator inhibitor-1 production inhibitor of the present invention can be orally or parenterally administered. As parenteral administration routes there are intravenous injection, subcutaneous injection, intramuscular injection, perintestinal administration, percutaneous administration, eye instillation and pernasal administration.

Regarding the administration dosage in the present invention, for adults the intracellular cyclic AMP increasing substance is administered 1-3 times per day at a dose of 0.1 µg - 500 mg/person.

With regard to the plasminogen activator inhibitor-1 production inhibitor of the present invention, one or several types of intracellular cyclic AMP increasing substance may be used directly as they are, but oral administration can also be carried out in the form of a solid material containing undermentioned additives.

As examples of additives there are starches, lactose, sucrose, glucose, mannitol, calcium carbonate, calcium sulphate and other such excipients; starches, dextrin, gum Arabic, tragacanth, methyl cellulose, gelatin, polyvinyl pyrrolidone, polyvinyl alcohol and other such binders; starches, polyvinyl pyrrolidone, crystalline cellulose and other such disintegrants; magnesium stearate, talc and other such lubricants; and colorants and perfumes, etc.

The plasminogen activator inhibitor-1 production inhibitor of the present invention can be used in various dosage forms; specifically, as tablets, sugar-coated tablets, powders, granules, troches, capsules, pills, syrups and other such conventionally-used dosage forms.

Furthermore, parenteral administration in the form of a sterilized solution may also be carried out and, moreover, other dissolved substances can also be employed, for example sufficient sodium chloride, glucose or the like, to make the solution isotonic.

As a specific example of a formulation, there is the following.
- intracellular cAMP increasing substance: 1 mg
- physiological saline: 1 ml

### Examples

Below, the present invention is explained in more specific terms by means of examples. However, the present invention is not restricted to the examples provided.

### Reference Example 1 Preparation of Materials.

### (1) AGE-BSA preparation

AGE-BSA was formed by incubating bovine serum albumin (BSA) (fraction V, fatty acid-free, endotoxin-free, produced by Boehringer Mannheim GmbH, Germany) with 0.5 M glucose at 37°C for 6 weeks under sterilized conditions. Unbound glucose was removed by dialyzing against phosphate-buffered saline (PBS) and the glucose-modified macromolecular substance was purified by heparin-Sepharose CL-4B column (produced by Pharmacia LKB, Uppsala, Sweden) chromatography and used as the AGE-BSA. Control non-glycated BSA was prepared by carrying out incubation under the same conditions as aforementioned, except for not including the glucose. The fact that non-glycated BSA had been separated from the AGE-BSA was confirmed by SDS-PAGE. The AGE-BSA concentration was measured by the Bradford method (Bradford, M.: Anal. Biochem. 72: 248-254, 1976).

### (2) Preparation of anti-AGE-RNaseA antiserum

Anti-AGE-RNaseA antiserum was prepared by a known method (Yamagishi, S. et al., J. Biol. Chem. 272: 8723-8730, 1997) and characterization was carried out.

### (3) Preparation of vascular endothelial cells

Endothelial cells derived from the microvessels of human skin were maintained in E-BM medium containing 5% fetal calf serum, 0.4% bovine brain extract, 10 ng/ml human epidermal growth factor and 1 µg/ml hydrocortisone. Cells which had been subcultured 5-10 times were used for the tests. AGE-BSA treatment was carried out in E-BM medium of the same composition as above, except for the fact that the epidermal growth factor and hydrocortisone were not included.

### Example 1

The vascular endothelial cells prepared in Reference Example 1 (3) were treated at 37°C for 24 hours with 0 µ g/ml or 50 µg/ml of AGE-BSA by the method described in Reference Example 1 (3), in the presence of various intracellular cAMP increasing substances. The intracellular cAMP increasing substances used were 1 µM Beraprost sodium (produced by Toray Industries, Inc., shown as PGI₂ in Figure 1), 10 µM forskolin (produced by the Sigma Co., shown as FOR in Figure 1) and 1 mM dibutyryl cAMP (produced by the Sigma Co., shown as db in Figure 1). Moreover, for comparison, tests were similarly carried out using the anti-AGE-RNaseA antiserum prepared in Reference Example 1 (2) (shown as Ab in Figure 1) at a concentration of 1 wt%. Again, for comparison, samples treated in the same way as above except for the fact that no intracellular cAMP increasing substance was included were also tested.

The amount of PAI-1 released into the medium was measured using a commercially available ELISA kit (Imulyse PAI-1, UMEA, Sweden) in accordance with the manufacturer's instructions. In this method, almost no PAI-1 which has complexed with urokinase-type plasminogen activator (u-PA) or t-PA is detected and so, in the present example, the free PAI-1 concentration could be selectively measured.

The results are shown in Figure 1. On the horizontal axis in Figure 1, the compounds used for treatment of the vascular endothelial cells and their concentrations are shown and the vertical axis shows the amounts of PAI-1 released into the medium per 10⁶ cells. The bars on the left of Figure 1 show the amounts of PAI-1 released by normal vascular endothelial cells which were not treated with AGE-BSA. When treated with 50 µg/ml of AGE-BSA (5^{th} bar from the left), the amount of PAI-1 released increased to approximately 130%. On the other hand, on the basis of separate tests it was confirmed that when treated with non-glycated BSA prepared in Reference Example 1 (1), the amount of PAI-1 released was not increased. Thus, it was confirmed that the level of PAI-1 release from vascular endothelial cells was increased by AGE. Furthermore the action of AGE was completely neutralized by the AGE-RNaseA antiserum but, since the same concentration of antiserum did not have an effect on the production of PAI-1 by endothelial cells which had not been exposed to AGE-BSA, it was concluded that the action of AGE on PAI-1 production is due to unique structures common to AGE. On the other hand, when treated at a concentration of 1 µM with Beraprost sodium (BPS) which is an intracellular cAMP increasing substance, the level of PAI-1 release decreased to the same level as the amount released by normal vascular endothelial cells which had not been treated with AGE-BSA. Furthermore, when treated with 10 µM forskolin (FOR) and 1 mM dibutyryl cAMP (db), the levels of PAI-1 release were further decreased from normal values. From these results it was clear that PAI-1 production by vascular endothelial cells was suppressed by intracellular cAMP increasing substances.

### Industrial Application Potential

By means of the PAI-1 production inhibitor of the present invention, cellular PAI-1 production is effectively suppressed. Consequently, the PAI-1 production inhibitor of the present invention is effective in thrombus degradation, and it is effective in the prevention and treatment of ischaemic heart disease such as angina pectoris, myocardial infarction and cardiac insufficiency, ischaemic cerebrovascular disorders such as cerebral embolism, cerebral infarction and transient ischaemic attack and various thromboses such as venous thrombosis of the vena cava and vena hepatica, pulmonary embolism and postoperative thrombosis, where thrombus formation is closely related to the onset and aggravation of the disease state. Moreover, it is particularly effective in the prevention and treatment of complications of diabetes comprising vascular disorders, microvascular disorders, diabetic neuropathy, retinopathy, nephropathy and ischaemic heart disease, where an increase in blood and accumulated AGE is closely related to an aggravation of the disease state.

## Claims

1. Plasminogen activator inhibitor-1 production inhibitor containing as the effective component an intracellular cyclic AMP increasing substance.

2. Plasminogen activator inhibitor-1 production inhibitor according to Claim 1 where the aforesaid cyclic AMP increasing substance is a prostaglandin compound.

3. Plasminogen activator inhibitor-1 production inhibitor according to Claim 2 where the aforesaid prostaglandin compound is a 4,8-inter-m-phenylene prostaglandin I₂ derivative represented by general formula (I) 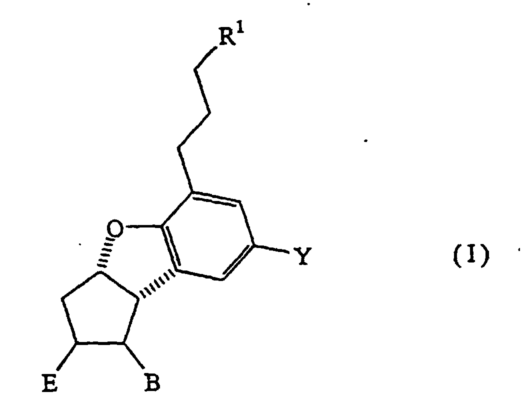 [where R¹ is
(A) COOR²
(where R² is
1) hydrogen or a pharmacologically acceptable cation,
2) a C₁₋₁₂ straight chain alkyl or c₃₋₁₄ branched alkyl,
3) -Z-R³
(where Z is a valence bond or a straight chain or branched alkylene represented by CₜH₂ₜ, where t denotes an integer in the range 1-6, and R³ is a C₃₋₁₂ cycloalkyl or C₃₋₁₂ substituted cycloalkyl which is substituted with from one to three R⁴ groups, and R⁴ denotes hydrogen or a C₁₋₅ alkyl),
4) -(CH₂CH₂O)ₙCH₃
(where n denotes an integer in the range 1 to 5),
5) -Z-Ar¹
(where Z is defined as above and Ar¹ denotes phenyl, α-naphthyl, β-naphthyl, 2-pyridyl, 3-pyridyl, 4-pyridyl, α-furyl, β-furyl, α-thienyl, β-thienyl or substituted phenyl (where the substituent groups are one or more of chlorine, bromine, fluorine, iodine, trifluoromethyl, C₁₋₄ alkyl, nitro, cyano, methoxy, phenyl, phenoxy, p-acetamido-benzamido, -CH=N-NH-C(=O)-NH₂, -NH-C(=O)-Ph, - NH-C(=O)-CH₃ and -NH-C(=O)-NH₂)),
6) -CₜH₂ₜCOOR⁴
(where CₜH₂ₜ and R⁴ are as defined above),
7) -CₜH₂ₜN(R⁴)₂
(where CₜH₂ₜ and R⁴ are as defined above),
8) -CH(R⁵)-C(=O)-R⁶
(where R⁵ denotes hydrogen or benzoyl, and R⁶ denotes phenyl, p-bromophenyl, p-chlorophenyl, p-biphenyl, p-nitrophenyl, p-benzamidophenyl or 2-naphthyl),
9) -CₚH₂ₚ-W-R⁷
(where W is -CH=CH-, -CH=CR⁷- or -C≡C-, and R⁷ is hydrogen or a C₁₋₃₀ straight chain or branched alkyl or aralkyl, and p denotes an integer in the range 1-5), or
10) -CH(CH₂OR⁸)₂
(where R⁸ denotes a C₁₋₃₀ alkyl or acyl),
(B) -CH₂OH
(C) -C(=O)N(R⁹)₂
where R⁹ represents hydrogen, a C₁₋₁₂ straight chain alkyl, C₃₋₁₂ branched alkyl, C₃₋₁₂ cycloalkyl, C₄₋₁₃ cycloalkylalkylene, phenyl, substituted phenyl (where the substituent groups are as defined in (A) 5) above), C₇₋₁₂ aralkyl or SO₂R¹⁰, and R¹⁰ represents a C₁₋₁₀ alkyl, C₃₋₁₂ cycloalkyl, phenyl, substituted phenyl (where the substituent groups are as defined in (A) 5) above), or a C₇₋₁₂ aralkyl, and the two R⁹ groups may be the same or different, but where one represents -SO₂R¹⁰ then the other R⁹ is not -SO₂R¹⁰), or
(D) -CH₂OTHP (where THP is a tetrahydropyranyl group),
Y is hydrogen, a C₁₋₄ alkyl, chlorine, bromine, fluorine, formyl, methoxy or nitro,
B is -X-C(R¹¹)(R¹²)OR¹³
(where R¹¹ is hydrogen or a C₁₋₄ alkyl, R¹³ is hydrogen, C₁₋₁₄ acyl, C₆₋₁₅ aroyl, tetrahydropyranyl, tetrahydrofuranyl, 1-ethoxyethyl or t-butyl,
X is
1) -CH₂-CH₂-
2) -CH=CH- or
3) -C≡C- and
R¹² represents
1) a C₁₋₁₂ straight chain alkyl or C₃₋₁₄ branched alkyl,
2) -Z-Ar²
(where Z is as defined above and Ar² represents phenyl, α-naphthyl, β-naphthyl or phenyl substituted with at least one of chlorine, bromine, fluorine, iodine, trifluoromethyl, C₁₋₄ alkyl, nitro, cyano, methoxy, phenyl or phenoxy),
3) -CₜH₂ₜOR¹⁴
(where CₜH₂ₜ is as defined above and R¹⁴ represents a C₁₋₆ straight chain alkyl, C₃₋₆ branched alkyl, phenyl, phenyl substituted with at least one of chlorine, bromine, fluorine, iodine, trifluoromethyl, C₁₋₄ alkyl, nitro, cyano, methoxy, phenyl or phenoxy, cyclopentyl, cyclohexyl, or cyclopentyl or cyclohexyl substituted with from one to four C₁₋₄ straight chain alkyls),
4) -Z-R³
(where Z and R³ are as defined above),
5) -CₜH₂ₜ-CH=C(R¹⁵)R¹⁶
(where CₜH₂ₜ is as defined above, and R¹⁵ and R¹⁶ each independently represent hydrogen, methyl, ethyl, propyl or butyl), or
6) -CᵤH₂ᵤ-C≡C-R¹⁷
(where u is an integer in the range 1-7, CᵤH₂ᵤ represents a straight chain or branched alkylene, and R¹⁷ represents a C₁₋₆ straight chain alkyl),
E represents hydrogen or -OR¹⁸ (where R¹⁸ represents a C₁₋₁₂ acyl, C₇₋₁₅ aroyl or R² (where R² is as defined above),
and where the general formula represents the d-form, l-form or dl-form],
or pharmacologically permissible salts thereof.

4. Plasminogen activator inhibitor-1 production inhibitor according to Claim 3 where the aforementioned 4,8-inter-m-phenylene derivative shown in general formula (I) is Beraprost or salt thereof.

5. Plasminogen activator inhibitor-1 production inhibitor according to any of Claims 1 to 4, which is for suppressing plasminogen activator inhibitor-1 overproduction induced by advanced glycation endproducts.

6. Plasminogen activator inhibitor-1 production inhibitor according to any of Claims 1 to 4 where the plasminogen activator inhibitor-producing cells are vascular endothelial cells.
